(19) Europäisches Patentamt
European Patent Office
Office européen des brevets

(11) Numéro de publication : **0 406 088 B1**

# (12) FASCICULE DE BREVET EUROPEEN

(45) Date de publication du fascicule du brevet : **16.03.94 Bulletin 94/11**

(51) Int. Cl.$^5$ : **C07C 323/60,** A61K 31/16, A23K 1/16

(21) Numéro de dépôt : **90401801.7**

(22) Date de dépôt : **25.06.90**

## (54) (Alfa-tertiobutyl aminométhyl-3,4-dichlorobenzyl) thioacétamide, son procédé de préparation et ses applications.

(30) Priorité : **26.06.89 FR 8908480**

(43) Date de publication de la demande : **02.01.91 Bulletin 91/01**

(45) Mention de la délivrance du brevet : **16.03.94 Bulletin 94/11**

(84) Etats contractants désignés : **AT BE CH DE DK ES FR GB GR IT LI LU NL SE**

(56) Documents cités : **EP-A- 0 158 545**

(73) Titulaire : **LABORATOIRE L. LAFON**
**19 avenue du Professeur Cadiot**
**F-94700 Maisons Alfort Cédex (FR)**

(72) Inventeur : **Lafon, Louis**
**5 rue de l'Alboni**
**F-75016 Paris (FR)**

(74) Mandataire : **Le Guen, Gérard et al**
**CABINET LAVOIX 2, place d'Estienne d'Orves**
**F-75441 Paris Cédex 09 (FR)**

EP 0 406 088 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue Saint-Denis, 75001 PARIS

## Description

La présente invention est relative à de nouveaux dérivés de l'acide α-aminométhyl-benzylthioacétique et en particulier à de nouveaux dérivés de l'α-aminométhyl-benzylthioacétamide.

On a déjà décrit des dérivés de l'acide α-aminométhyl-benzylthioacétique dans EP-A-O-158 545. Ces composés ont une action sur le système nerveux central et peuvent être utilisés en thérapeutique comme antidépresseurs.

On a maintenant trouvé des dérivés de l'acide α-aminométhyl-benzylthioacétique qui présentent une action nettement supérieure sur le système nerveux central.

La présente invention a en conséquence pour objet le [α-(tert-butyl aminométhyl)-3,4-dichloro benzyl] thio acétamide de formule :

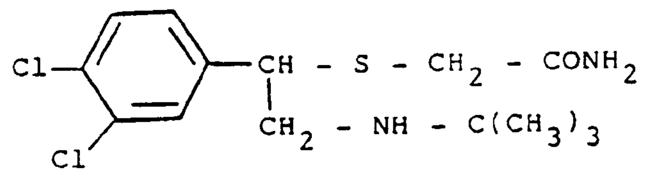

(I)

et ses sels d'addition avec des acides pharmaceutiquement acceptables.

Les "sels d'addition avec des acides pharmaceutiquement acceptables" désignent les sels qui donnent les propriétés biologiques des bases libres, sans avoir d'effet indésirable. Ces sels peuvent être notamment ceux formés avec des acides minéraux, tels que l'acide chlorhydrique, l'acide bromhydrique, l'acide sulfurique, l'acide nitrique, l'acide phosphorique; des sels métalliques acides, tels que l'orthophosphate disodique et le sulfate monopotassique, et des acides organiques, tels que l'acide formique, l'acide acétique, l'acide propionique, l'acide glycolique, l'acide oxalique, l'acide fumarique, l'acide maléique, l'acide citrique, l'acide malonique, l'acide méthane sulfonique, l'acide lactique, l'acide succinique, l'acide tartrique.

Le composé de formule I peut être obtenu par réaction du 1-(3,4-dichlorophényl)-2-bromo-éthanol avec la tert. butylamine, transformation de l'amino-alcool obtenu en dérivé amino chloré, condensation du dérivé amino chloré avec le thioglycolate de méthyle et aminolyse de l'ester ainsi obtenu.

L'exemple suivant illustre la préparation des composés selon l'invention.

### Exemple

Préparation du chlorhydrate de l'[α-(tert. butylaminométhyl)-3,4-dichlorobenzyl/thioacétamide (CRL 41 414).

1) Préparation du chlorhydrate du 2-(tert. butylamino)-1-(3,4 dichlorphényl)éthanol.

60 g (0,22 mole) de 1-(3,4-dichlorophényl)-2-bromoéthanol en solution dans 200 ml d'éthanol, sont ajoutés à une solution de 80 ml de tertiobutylamine dans 200 ml d'éthanol. Après 48 heures à 20° C et 3 heures à reflux, on évapore à sec sous vide, reprend avec NaOH N, extrait à l'éther, extrait celui-ci avec HCl 2N, précipite avec NaOH concentré, essore, lave à l'eau, sèche. On obtient la base (F = 111° C) avec un rendement de 80 %; celle-ci en solution dans l'acétate d'éthyle est transformée quantitativement en chlorhydrate par addition d'éthanol chlorhydrique (F = 210 - 211° C).

2) Préparation du chlorhydrate du 2-(tert. butylamino)-1-(3,4-dichlorophényl)1-chloroéthane.

29,85 g (0,1 mole) du chlorhydrate obtenu en 1) dans 120 ml de $CH_2Cl_2$ sont traités avec 20 ml de $SOCl_2$ dans 50 ml de $CH_2Cl_2$; après 5 heures à reflux et une nuit au repos, on essore, lave à l'éther, sèche et obtient le dérivé chloré, (F = 236-238° C) avec un rendement quantitatif.

3) Préparation du chlorhydrate de l'[α(tert. butylaminométhyl)-3,4-dichlorobenzyl] thioacétate de méthyle.

On mélange à froid 4,4 g (0,192 Atg) de sodium dans 250 ml de méthanol, puis 10 ml (0,1 mole) de thioglycolate de méthyle et 30,5 g (0,096 mole) du chlorhydrate du dérivé chloré obtenu en 2). Après 1/2 heure à 20° C, on chauffe 3 heures à reflux. On filtre le NaCl, évapore le filtrat sous vide, reprend à l'éther, lave à l'eau, extrait avec 100 ml d'HCl N, précipite à froid avec NaOH concentré, extrait à l'éther, lave à l'eau, sèche, filtre,

acidifie avec de l'éthanol chlorhydrique, essore, lave à l'éther et à l'acétate d'éthyle et sèche. On obtient le chlorhydrate de l'ester (F = 154-155° C) avec un rendement de 68 %.

4) Préparation du chlorhydrate de l'[α-(tert. butylaminométhyl)-3,4-dichlorobenzyl] thioacétamide.

15,9 g (0,04 mole) du chlorhydrate obtenu en (3) en solution dans 200 ml de méthanol sont traités avec 80 ml d'ammoniaque à 28 %.

Après 24 heures en contact, on évapore l'alcool, ajoute 50 ml d'HCl N, filtre sur charbon, précipite avec NaOH concentré, extrait à l'éther, sèche, filtre et acidifie avec de l'éthanol chlorhydrique, essore, lave à l'acétate d'éthyle et recristallise dans l'acétone.

On obtient le composé avec un rendement de 42 %.

C'est une poudre blanche, soluble dans l'eau et les alcools ; insoluble dans l'éther, l'acétate d'éthyle.

Il fond à 176° C.

On donnera ci-après des résultats pharmacologiques et toxicologiques mettant en évidence les avantages du composé (CRL 41414) selon l'invention par rapport aux composés décrits dans EP-A-O 158 545.

Comme composé de comparaison, on a utilisé le composé qui a été décrit dans EP-A-O 158 545 et qui est structurellement le plus proche des composés selon l'invention, à savoir le fumarate de l'[α-(isopropylaminométhyl)-3,4-dichlorobenzyl] thioacétamide (CRL 41253).

1) Toxicité

Une étude de prétoxicité a été réalisée chez la souris par voie i.p.

a) CRL 41 414

Pas de mortalité à 128 mg/kg. A 256 mg/kg, on observe des convulsions entraînant la mort en 15 minutes (3 animaux sur 3).

b) CRL 41 253

Pas de mortalité à 256 mg/kg mais convulsions pour 1 souris sur 3. A 512 mg/kg, on observe une sédation puis des convulsions entraînant la mort (3 animaux sur 3).

Le CRL 41 253 est donc au mieux deux fois moins toxique que le CRL 41 414.

2) Antagonisme des hypothermies induites par l'apomorphine, la réserpine et l'oxotrémorine chez la souris (administration i.p. des composés testés)

| | CRL 41 414 | CRL 41 253 |
|---|---|---|
| Apomorphine 16 mg/kg | 1 mg/kg +++ | 32 mg/kg + |
| Réserpine 2,5 mg/kg | 1 mg/kg + | 32 mg/kg + |
| Oxotrémorine 0,5 mg/kg | 1 mg/kg + | 32 mg/kg +. |

(Dans ce tableau, l'effet + correspond à un début d'effet alors que l'effet +++ est un effet très net).

Ces résultats mettent en évidence l'activité du CRL 41 414 à des doses 32 fois plus faibles que le CRL 41 253 dans ces tests qui sont des tests classiques pour la mise en évidence d'un effet antidépresseur.

3) Action sur le désespoir comportemental.

Une demi-heure après avoir administré le produit testé par voie i.p. à des lots de 6 souris, on place des souris dans un bécher rempli d'eau. On détermine la diminution de l'immobilité sous l'effet du produit testé.

| | CRL 41 414 | CRL 41 253 |
|---|---|---|
| Dose minimale diminuant la durée d'immobilité | 4 mg/kg | 32 mg/kg. |

Les résultats mettent en évidence un effet du CRL 41 414 à des doses 8 fois plus faibles dans ce test qui confirme l'effet antidépresseur du CRL 41 414.

4) Action sur le sommeil barbiturique.

Une demi-heure après l'administration du produit testé par voie i.p., des lots de souris reçoivent une injection intrapéritonéale de barbital (220 mg/kg).

On détermine la dose minimale qui diminue la durée du sommeil barbiturique.

| | CRL 41 414 | CRL 41 253 |
|---|---|---|
| Dose minimale | 4 mg/kg | 32 mg/kg. |

Ce test met en évidence un effet stimulant à la dose de 4 mg/kg pour le composé selon l'invention.

La présente invention a également pour objet des compositions thérapeutiques comprenant à titre de principe actif l'[α-(tert. butyl aminométhyl)-3,4-dichlorobenzyl]thioacétamide ou l'un de ses sels d'addition avec des acides pharmaceutiquement acceptables.

Les compositions thérapeutiques selon l'invention peuvent être administrées à l'homme ou aux animaux par voie orale ou parentérale.

Elles peuvent être sous la forme de préparations solides, semi-solides ou liquides. Comme exemple, on peut citer les comprimés, les gélules, les suppositoires, les solutions ou suspensions injectables, ainsi que les formes-retard et les formes implantées à libération lente.

Dans ces compositions, le principe actif est généralement mélangé avec un ou plusieurs excipients pharmaceutiquement acceptables habituels bien connus de l'homme de l'art.

La quantité de principe actif administrée dépend évidemment du patient qui est traité, de la voie d'administration et de la sévérité de la maladie.

On a par ailleurs découvert que le composé selon l'invention favorisait la prise alimentaire chez l'animal et pouvait donc être utilisé pour stimuler l'appétit chez l'homme et les animaux.

On donnera ci-après des résultats d'une étude réalisée chez le rat sur l'effet du composé selon l'invention sur la prise alimentaire.

Pour l'étude, on a utilisé des rats mâles Sprague Dawley âgés de 80 jours et pesant en moyenne 374 g le premier jour des mesures. Ces rats ont été placés en cages individuelles, 22 jours avant le début des mesures. Les animaux ont été nourris ad libitum avec le régime Extralabo M2O et ont disposé en permanence d'eau à volonté. Les animaux ont été conditionnés à un cycle lumineux comprenant 8 h d'obscurité et 16 h de lumière. Le composé testé, en solution dans du sérum physiologique (NaCl à 9/1000), ainsi qu'un placebo constitué par du sérum physiologique, ont été administrés par voie intrapéritonéale sous un volume de 1 ml/kg.

Les résultats sont représentés sur la Fig. unique annexée sur laquelle on a reporté en fonction du temps la variation de la prise alimentaire en % de la prise alimentaire sous placebo.

Sur cette Fig., la courbe A correspond à la dose de 0,25 mg/kg, la courbe B à la dose de 0,50 mg/kg et la courbe C à la dose de 1 mg/kg.

On constate avec le composé de formule I une augmentation de la prise alimentaire qui est de 7 à 11 fois supérieure à celle observée sans l'administration du composé de formule I.

La présente invention a donc également pour objet une composition favorisant la prise alimentaire chez l'homme et les animaux et qui comprend à titre de principe actif le composé de formule I. La présente invention trouve une application notamment dans l'alimentation des animaux d'élevage tels que le bétail ou la volaille où la composition selon l'invention permet d'obtenir notamment une augmentation de la prise pondérale.

Pour l'alimentation animale la composition peut prendre notamment la forme d'un prémélange contenant le principe actif dispersé dans un véhicule ou diluant ou encore la forme d'un supplément de nourriture contenant le principe actif mélangé à un véhicule ou diluant.

Dans ces deux derniers cas, le véhicule est de préférence un composant alimentaire pour animaux, par exemple avoine, graine de soja, luzerne, froment, résidus de fermentation, coquilles d'huître broyées, mélasses, substances végétales comestibles, farine de soja, kaolin, talc, calcaire broyé, etc ...

Sous forme de prémélange, des compositions selon l'invention destinées à favoriser l'alimentation animale peuvent contenir de 10 à 80 % en poids de composé de formule I ou d'un de ses sels non toxiques et de 90 à 20 % de véhicule ou diluant en poids de la composition. Ces prémélanges peuvent être dilués avec un supplément alimentaire pour animaux ou peuvent être ajoutés directement à une ration alimentaire animale de manière à fournir une alimentation appropriée.

Alternativement, le composé de formule I et ses sels non toxiques peuvent être administrés sous forme d'une solution ou suspension contenant une quantité efficace de ce principe actif dans l'eau de boisson des animaux.

Une telle quantité favorable peut varier de 0,0005 à 0,05 % en poids de la quantité de nourriture journalière chez les animaux.

## Revendications

### Revendications pour les Etats contractants suivants : AT, BE, CH, DE, DK, FR, GB, GR, IT, LI, LU, NL, SE

1. [α-(tert-butyl aminométhyl)-3,4-dichloro benzyl] thio acétamide de formule :

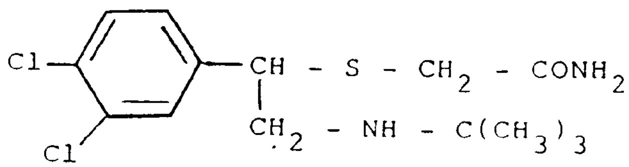

(I)

et ses sels d'addition avec des acides pharmaceutiquement acceptables.

2. Procédé de préparation d'un composé selon la revendication 1, caractérisé en ce que l'on fait réagir le 1-(3,4-dichlorophényl) 2-bromoéthanol avec la tert. butylamine, on transforme l'aminoalcool obtenu en dérivé amino-chloré, on condense le dérivé aminochloré avec le thioglycolate de méthyle et on effectue une aminolyse de l'ester ainsi obtenu.

3. Composition thérapeutique comprenant, à titre de principe actif, un composé selon la revendication 1.

4. Composition favorisant la prise alimentaire comprenant à titre de principe actif un composé selon la revendication 1.

5. Composition pour l'alimentation des animaux, comprenant un composé selon la revendication 1 en une quantité efficace pour favoriser la prise alimentaire.

6. Procédé pour favoriser la prise alimentaire chez les animaux, qui comprend l'addition d'un composé selon la revendication 1 en une quantité efficace pour favoriser la prise alimentaire, à l'alimentation des animaux.

### Revendications pour l'Etat contractant suivant : ES

1. Procédé de préparation du [α-(tert-butyl aminométhyl)-3,4-dichlorobenzyl] thio acétamide de formule :

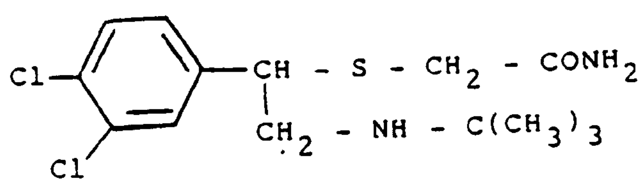

(I)

et de ses sels d'addition avec des acides pharmaceutiquement acceptables, caractérisé en ce que l'on fait réagir le 1-(3,4-dichlorophényl)-2-bromoéthanol avec la tert. butylamine, on transforme l'aminoalcool obtenu en dérivé amino-chloré, on condense le dérivé aminochloré avec le thioglycolate de méthyle et on effectue une aminolyse de l'ester ainsi obtenu.

2. Composition favorisant la prise alimentaire comprenant à titre de principe actif l'[α-tert-butyl aminométhyl)3-4-dichlorobenzyl] thio acétamide de formule :

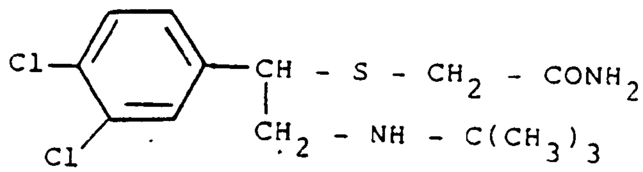

(I)

ou l'un de ses sels d'addition avec des acides pharmaceutiquement acceptables.

**3.** Composition pour l'alimentation des animaux comprenant l'[α-(tert-butyl aminométhyl)-3,4-dichlorobenzyl] thio acétamide de formule :

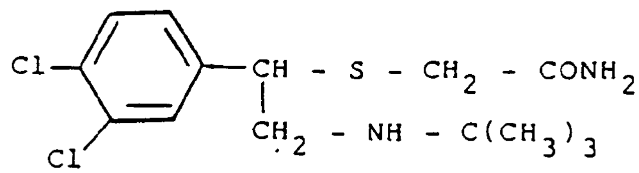

(I)

ou l'un de ses sels d'addition avec des acides pharmaceutiquement acceptables en une quantité efficace pour favoriser la prise alimentaire.

**4.** Procédé pour favoriser la prise alimentaire chez les animaux, qui comprend l'addition d'[α-(tert-butyl aminométhyl)-3,4-dichlorobenzyl] thio acétamide de formule :

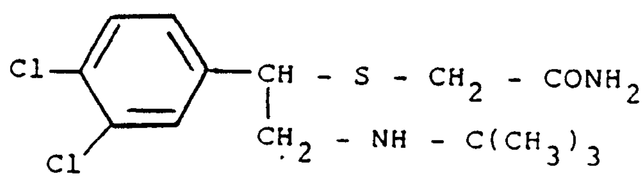

(I)

ou l'un de ses sels d'addition avec des acides pharmaceutiquement acceptables, en une quantité efficace pour favoriser la prise alimentaire, à l'alimentation des animaux.

**Claims**

**Claims for the following Contracting States : AT, BE, CH, DE, DK, FR, GB, GR, IT, LI, LU, NL, SE**

**1.** [α-(tert-Butylaminomethyl)-3,4-dichlorobenzyl]-thioacetamide of formula:

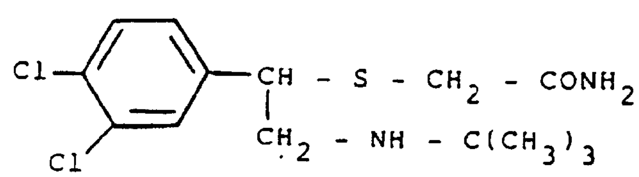

(I)

and its addition salts with pharmaceutically acceptable acids.

**2.** Process for preparing a compound according to Claim 1, characterized in that 1-(3,4-dichlorophenyl)-2-bromoethanol is reacted with tert-butylamine, the amino alcohol obtained is converted to a chlorinated amino derivative, the chlorinated amino derivative is condensed with methyl thioglycolate and an aminolysis of the ester thereby obtained is performed.

**3.** Therapeutic composition comprising a compound according to Claim 1 as active principle.

4. Composition promoting feed intake, comprising a compound according to Claim 1 as active principle.

5. Animal feed composition, comprising a compound according to Claim 1 in an effective amount for promoting feed intake.

6. Process for promoting feed intake in animals, which comprises the addition of a compound according to Claim 1, in an effective amount for promoting feed intake, to animal feed.

**Claims for the following Contracting State : ES**

1. Process for preparing [α-(tert-Butylaminomethyl)-3,4-dichlorobenzyl]thioacetamide of formula:

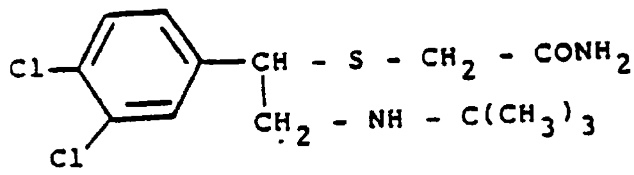

(I)

and its addition salts with pharmaceutically acceptable acids, characterized in that 1-(3,4-dichlorophenyl)-2-bromoethanol is reacted with tert-butylamine, the amino alcohol obtained is converted to a chlorinated amino derivative, the chlorinated amino derivative is condensed with methyl thioglycolate and an aminolysis of the ester thereby obtained is performed.

2. Composition promoting feed intake, comprising as active principle [α-(tert-butylaminomethyl)-3,4-dichlorobenzyl]thioacetamide of formula:

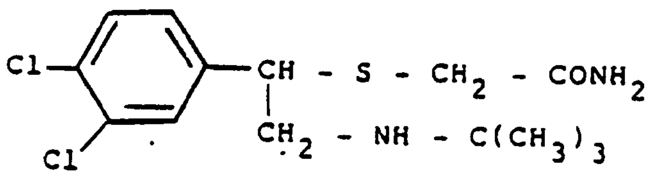

(I)

or one of its addition salts with pharmaceutically acceptable acids.

3. Animal feed composition, comprising [α-(tert-butylaminomethyl)-3,4-dichlorobenzyl]thioacetamide of formula:

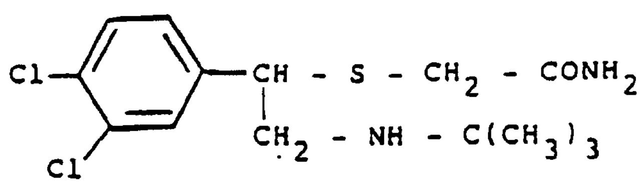

(I)

or one of its addition salts with pharmaceutically acceptable acids, in an effective amount for promoting feed intake.

4. Process for promoting feed intake in animals, which comprises the addition of [α-(tert-butylaminomethyl)-3,4-dichlorobenzyl]thioacetamide of formula:

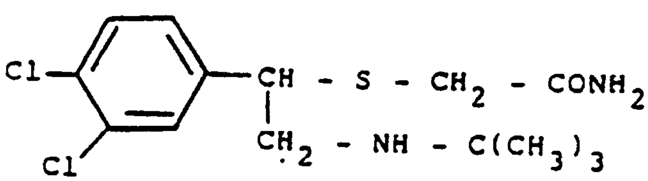

(I)

or one of its addition salts with pharmaceutically acceptable acids, in an effective amount for promoting feed intake, to animal feed.

## Patentansprüche

### Patentansprüche für folgende Vertragsstaaten : AT, BE, CH, DE, DK, FR, GB, GR, IT, LI, LU, NL, SE

1. [α-(tert.-Butyl-aminomethyl)-3,4-dichlorbenzyl]-thioacetamid der Formel:

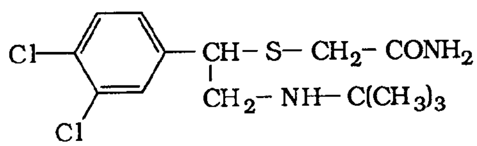

(I)

und dessen Additionssalze mit pharmazeutisch annehmbaren Säuren.

2. Verfahren zur Herstellung einer Verbindung nach Anspruch 1, **dadurch gekennzeichnet**, daß man 1-(3,4-Dichlorphenyl)-2-bromethanol mit tert.-Butylamin umsetzt, den erhaltenen Aminoalkohol in das Amino-Chlor-Derivat umwandelt, das Amino-Chlor-Derivat mit Thioglykolsäuremethylester kondensiert und eine Aminolyse des in dieser Weise erhaltenen Esters bewirkt.

3. Therapeutische Zubereitung enthaltend als Wirkstoff eine Verbindung nach Anspruch 1.

4. Zubereitung zur Begünstigung der Nahrungsaufnahme enthaltend als Wirkstoff eine Verbindung nach Anspruch 1.

5. Zubereitung zur Fütterung von Tieren enthaltend eine Verbindung nach Anspruch 1 in einer zur Begünstigung der Futteraufnahme ausreichenden Menge.

6. Verfahren zur Begünstigung der Futteraufnahme bei Tieren, welches darin besteht, eine Verbindung nach Anspruch 1 in einer zur Begünstigung der Futteraufnahme wirksamen Menge dem Futter der Tiere beizumengen.

### Patentansprüche für folgenden Vertragsstaat : ES

1. Verfahren zur Herstellung von [α-(tert.-Butyl-aminomethyl)-3,4-dichlorbenzyl]-thioacetamid der Formel:

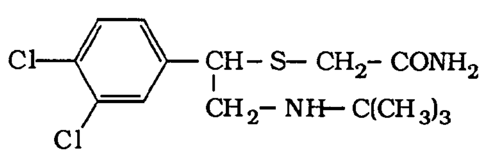

(I)

und dessen Additionssalze mit pharmazeutisch annehmbaren Säuren, **dadurch gekennzeichnet**, daß man 1-(3,4-Dichlorphenyl)-2-bromethanol mit tert.-Butylamin umsetzt, den erhaltenen Aminoalkohol in das Amino-Chlor-Derivat umwandelt, das Amino-Chlor-Derivat mit Thioglykolsäuremethylester kondensiert und eine Aminolyse des in dieser Weise erhaltenen Esters bewirkt.

2. Zubereitung zur Begünstigung der Nahrungsaufnahme enthaltend als Wirkstoff [α-(tert.-Butyl-aminomethyl)-3,4-dichlorbenzyl]-thioacetamid der Formel:

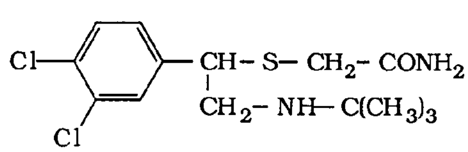

(I)

oder eines seiner Additionssalze mit pharmazeutisch annehmbaren Säuren.

**3.** Zubereitung zur Fütterung von Tieren enthaltend [α-(tert.-Butyl-aminomethyl)-3,4-dichlorbenzyl]-thioacetamid der Formel:

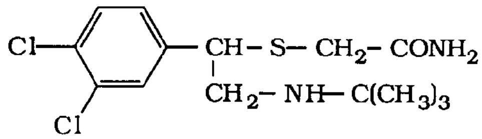

(I)

oder eines seiner Additionssalze mit pharmazeutisch annehmbaren Säuren in einer zur Begünstigung der Futteraufnahme wirksamen Menge.

**4.** Verfahren zur Begünstigung der Futteraufnahme bei Tieren, welches darin besteht, [α-(tert.-Butyl-aminomethyl)-3,4-dichlorbenzyl]-thioacetamid der Formel:

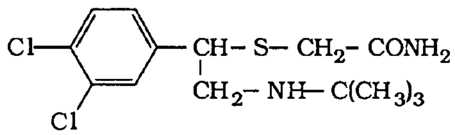

(I)

oder eines seiner Additionssalze mit pharmazeutisch annehmbaren Säuren in einer zur Begünstigung der Futteraufnahme wirksamen Menge dem Tierfutter beizumengen.

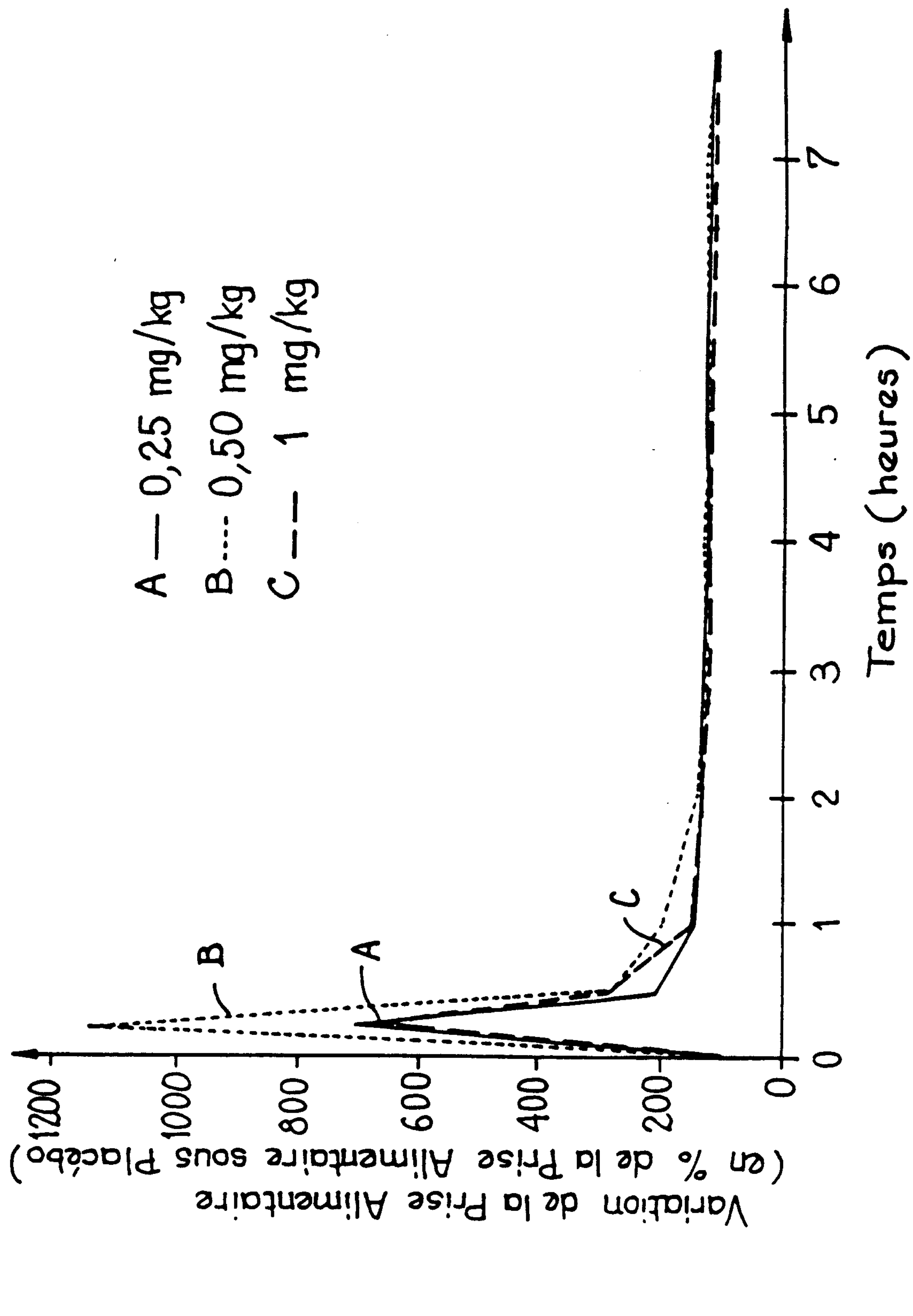
A —— 0,25 mg/kg
B ···· 0,50 mg/kg
C — — 1 mg/kg
A
B
C
Temps (heures)
0
1
2
3
4
5
6
7
Variation de la Prise Alimentaire
(en % de la Prise Alimentaire sous Placébo)
0
200
400
600
800
1000
1200